# DEMANDE DE BREVET EUROPEEN

(11) **EP 0 787 484 A1**
(43) Date de publication de la demande: **06.08.1997**
(21) Numéro de dépôt: 97400156.2
(22) Date de dépôt: 23.01.1997
(51) Int. Cl.: A61K 7/48, A61K 7/06

(54) **Utilisation d'extrait d'algue macroscopique comme transporteur d'oxygene**

(30) Priorité: 30.01.1996 FR 9601087
(71) Demandeur: L'OREAL, 75008 Paris (FR)
(72) Inventeur: Nguyen, Quang Lan, 92160 Antony (FR); Millecamps, François, 91270 Vigneux Sur Seine (FR)
(74) Mandataire: Lhoste, Catherine

(57) **Abrégé**

La présente invention se rapporte à l'utilisation, dans les compositions cosmétiques ou dermatologiques, d'un extrait d'une algue macroscopique, de poids moléculaire inférieur à 10000 daltons, comme transporteur d'oxygène dans la peau. Cet extrait est en particulier la SPD ou la Superphyco-D.

Les compositions selon l'invention sont notamment utilisées pour tonifier la peau et le cuir chevelu et pour empêcher la chute des cheveux.

## Description

La présente invention se rapporte à l'utilisation, dans les compositions cosmétiques ou dermatologiques, d'un extrait d'une algue macroscopique, de poids moléculaire inférieur à 10000 daltons, comme transporteur d'oxygène dans la peau et le cuir chevelu, et plus particulièrement à son application pour tonifier la peau et le cuir chevelu. L'utilisation de cet extrait permet également d'empêcher la chute des cheveux.

La peau, y compris celle du cuir chevelu, comme les autres organes de l'organisme, a besoin d'être régulièrement oxygénée pour bien fonctionner. En effet, il est connu que l'oxygène est nécessaire à la synthèse de l'A.T.P. (adénosine triphosphate), source d'énergie pour les cellules de l'organisme, et que le manque d'oxygène produit un disfonctionnement du métabolisme énergétique des cellules. En revanche, un apport en oxygène stimule ce métabolisme et permet d'améliorer l'état des cellules et notamment celles de la peau. Il s'ensuit une régénération cellulaire et une amélioration de l'état de la peau qui respire la santé. En particulier, on constate une diminution de certaines rides fines ou ridules du visage.

Toutefois, un trop grand apport d'oxygène dans la peau peut entraîner la formation d'un milieu proradicalaire favorisant la libération d'ions superoxydes. Or, on sait que l'ion superoxyde O₂· (oxygène actif) est un ion-radical dont la réactivité en fait un composé toxique, car il engendre, notamment en présence d'ions métalliques, des radicaux libres hydroxyle (OH·) hautement nocifs. En conséquence, il est nécessaire que l'apport en oxygène dans la peau soit contrôlé.

Pour apporter de l'oxygène à la peau, il a déjà été proposé d'utiliser des peroxydes qui libèrent de l'oxygène naissant, et notamment le peroxyde d'hydrogène. Toutefois, les peroxydes présentent des effets secondaires, en particulier des effets irritants, qui rendent leur utilisation redhibitoire dans les domaines cosmétique et dermatologique.

En outre, il est connu des documents EP-A-296661, WO-A-94/00098 et WO-A-94/09754 des compositions topiques à base de dérivés fluorocarbonés conduisant à la libération d'oxygène dans la peau. Ces dérivés sont des produits de synthèse, qui, comme tout composé de synthèse, peuvent s'accumuler dans les tissus en raison de la lenteur de leur cinétique de décomposition au contact de la peau, et entraîner indirectement un apport excessif d'oxygène avec les inconvénients en découlant évoqués ci-dessus.

Aussi, il subsiste le besoin d'un composé d'origine naturelle qui permette un apport contrôlé d'oxygène à la peau tout en évitant les inconvénients mentionnés précédemment.

La demanderesse a maintenant trouvé de manière inattendue que l'utilisation d'un extrait d'une algue macroscopique, de poids moléculaire inférieur à 10000 daltons dans une composition topique conduit à un apport contrôlé d'oxygène dans la peau et le cuir chevelu, sans les effets secondaires de l'art antérieur.

Aussi, la présente invention se rapporte à l'utilisation dans une composition cosmétique comportant un milieu cosmétiquement acceptable, d'au moins un extrait d'au moins une algue macroscopique, de poids moléculaire inférieur à 10000 daltons comme transporteur d'oxygène.

La présente invention a aussi pour objet l'utilisation d'au moins un extrait d'au moins une algue macroscopique, de poids moléculaire inférieur à 10000 daltons comme transporteur d'oxygène pour la fabrication d'une composition dermatologique comportant un milieu dermatologiquement acceptable.

Certes, il est connu d'introduire dans les compositions cosmétiques ou dermatologiques des extraits d'algue macroscopique. Ainsi, le document FR-A-2655268 décrit l'utilisation d'extraits d'algue macroscopique à activité antiradicalaire, dans des compositions pharmaceutiques et cosmétiques en vue de protéger le derme. Ces extraits peuvent être aussi utilisés dans des compositions alimentaires ou à usage agricole.

Cependant, ce document ne décrit ni ne suggère que ces extraits puissent oxygéner et tonifier la peau.

Or, la demanderesse a trouvé de manière inattendue que les extraits d'algues macroscopiques, de poids moléculaire inférieur à 10000 daltons présentent la propriété de capter l'oxygène à pH basique et de le relarger quand le pH devient plus acide. Cette propriété est de manière avantageuse mise à profit pour le traitement de la peau et du cuir chevelu, où l'épiderme présente un gradient de pH qui diminue d'environ 7,5 à 4,5 lorsque l'on va de la jonction dermo-épidermique à la surface de la peau. L'extrait selon l'invention, appliqué sur la peau, apporte de l'oxygène aux cellules à la peau et le transporte là où c'est nécessaire, à savoir jusqu'aux couches profondes de la peau, où cet oxygène est stocké. Lorsque se produit le renouvellement épidermique, entraînant la montée des cellules des couches profondes vers le *stratum comeum*, le pH des cellules devient plus acide et l'oxygène stocké par les cellules est alors relargé dans la peau. L'oxygène ainsi relargé se transforme en eau sous l'effet des oxydases de la peau, conduisant par conséquent à un meilleur tonus de la peau ou du cuir chevelu et assurant le bon fonctionnement des enzymes cutanées.

Les extraits d'algues utilisables dans la présente invention sont ceux ayant un poids moléculaire inférieur à 10000 daltons. Du fait de leurs faibles poids moléculaires, ces extraits vont pouvoir mieux pénétrer dans la peau ou le cuir chevelu que ceux ayant un poids moléculaire supérieur à 10000 daltons.

Selon une réalisation préférée de l'invention, l'extrait utilisé est choisi parmi les produits vendus sous les dénominations « SPD » et « Superphyco-D » par la société Secma. Ces extraits d'algue marine macroscopique sont bien tolérés par la peau et la cuir chevelu et ont par ailleurs des propriétés antiradicalaires, également bénéfiques pour la peau.

La présente invention a en outre pour objet un procédé cosmétique et/ou dermatologique pour oxygéner la peau et/ou le cuir chevelu, caractérisé en ce que l'on applique sur la peau et/ou le cuir chevelu, une composition cosmétique et/ou dermatologique contenant au moins un extrait d'au moins une algue macroscopique, de poids moléculaire inférieur à 10000 daltons.

La présente invention a encore pour objet un procédé cosmétique et/ou dermatologique pour tonifier la peau et/ou le cuir chevelu, caractérisé en ce que l'on applique sur la peau et/ou le cuir chevelu, une composition cosmétique et/ou dermatologique contenant au moins un extrait d'au moins une algue macroscopique, de poids moléculaire inférieur à 10000 daltons.

Une meilleure oxygénation du cuir chevelu contribue à améliorer son état général et notamment à empêcher la chute des cheveux.

Aussi, la présente invention a encore pour objet un procédé cosmétique et/ou dermatologique pour empêcher la chute des cheveux, caractérisé en ce que l'on applique sur le cuir chevelu une composition cosmétique et/ou dermatologique contenant au moins un extrait d'au moins une algue macroscopique, de poids moléculaire inférieur à 10000 daltons.

L'extrait selon l'invention peut, par exemple, être utilisé dans une composition cosmétique et/ou dermatologique selon l'invention en une concentration allant de 0,05 à 30 % en poids, et de préférence de 0,1 à 20 % en poids, et encore mieux de 0,5 à 10 % en poids par rapport au poids total de la composition.

Les compositions selon l'invention contiennent un milieu cosmétiquement et/ou dermatologiquement acceptable, c'est-à-dire compatible avec la peau, les muqueuses, les cheveux et le cuir chevelu. Elles peuvent se présenter sous toutes les formes galéniques appropriées pour une application topique, notamment sous forme de solutions aqueuses, hydroalcooliques ou huileuses, de gels aqueux, hydroalcooliques ou huileux, d'émulsions obtenues par dispersion d'une phase grasse dans une phase aqueuse (H/E) ou inversement (E/H), de suspensions, de microémulsions, de microcapsules, de microparticules, ou encore de dispersions vésiculaires de type ionique et/ou non ionique. Ces compositions sont préparées selon les méthodes usuelles dans les domaines considérés.

Les compositions selon l'invention peuvent être également utilisées sous forme de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains, pour les pieds ou pour le corps, des laits corporels de protection ou de soin, des lotions, gels ou mousses pour le soin de la peau et des cheveux, des compositions pour le bain, des compositions anti-chute pour le cuir chevelu.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique, pharmaceutique ou dermatologique. Les émulsionnants et coémulsionnants sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

De façon connue, la composition de l'invention peut contenir également des adjuvants habituels dans les domaines cosmétique, pharmaceutique et dermatologique, tels que les gélifiants, les actifs, les conservateurs, les antioxydants, les complexants tels que l'acide éthylènediamine tétracétique (EDTA) et les dérivés d'acide phosphonique, les solvants, les parfums, les tampons, les charges, les filtres, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer les huiles minérales (huile de vaseline), les huiles végétales, les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras (alcool cétylique), des acides gras, des cires (paraffine, carnauba, cire d'abeilles).

Comme émulsionnants utilisables dans l'invention, on peut citer par exemple le stéarate de glycérol et le Polysorbate 60.

Comme solvants utilisables dans l'invention, on peut citer les alcools inférieurs, notamment l'alcool éthylique et l'isopropanol.

Comme gélifiants hydrophiles, on peut citer les polymères carboxyvinyliques (carbomer), les polyacrylamides, les polysaccharides.

Comme actifs, on peut citer par exemple les polyols (glycérine, propylène glycol), et, dans les compositions pour le traitement de la chute des cheveux, le minoxidil.

Quand la composition contient des vésicules, les lipides principaux pouvant constituer ces vésicules sont choisis dans le groupe comprenant les lipides ioniques et les lipides non ioniques. Comme lipides non-ioniques on peut citer notamment les alkyléthers ou alkylesters de polyols comportant une ou plusieurs chaînes grasses par molécule, tels que l'éther de lauryl polyglycéryl-6-cétéaryl glycol, commercialisé par la société Chimex sous le nom Chimexane NS.

On peut de façon connue incorporer dans la phase lipidique constituant la membrane lipidique des vésicules, au moins un additif choisi par exemple parmi les lipoaminoacides et leurs sels tels que les acylglutamates de sodium, en particulier celui commercialisé sous la dénomination HS21 par la société Ajinomoto.

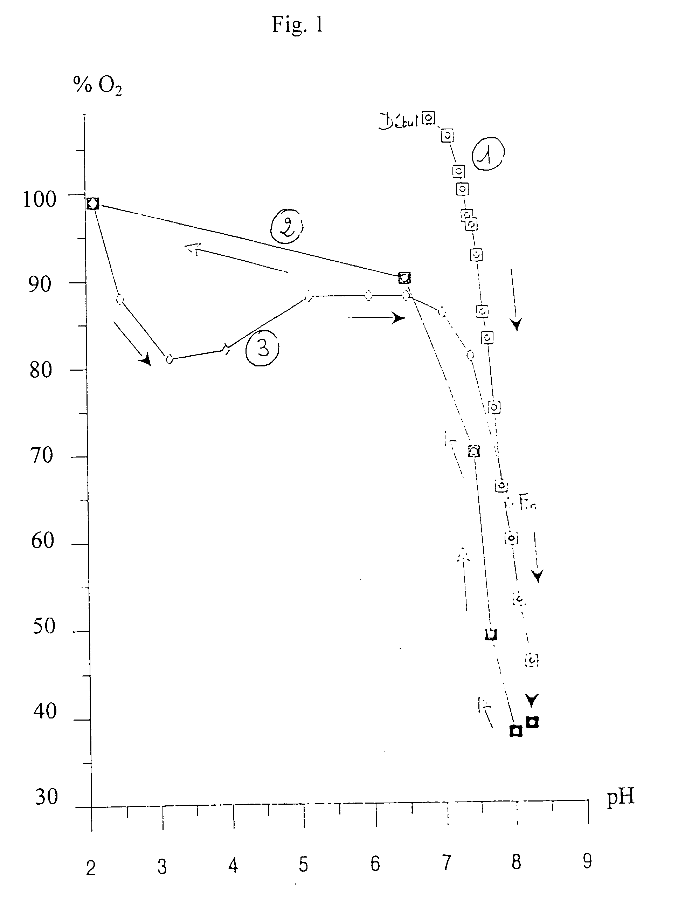

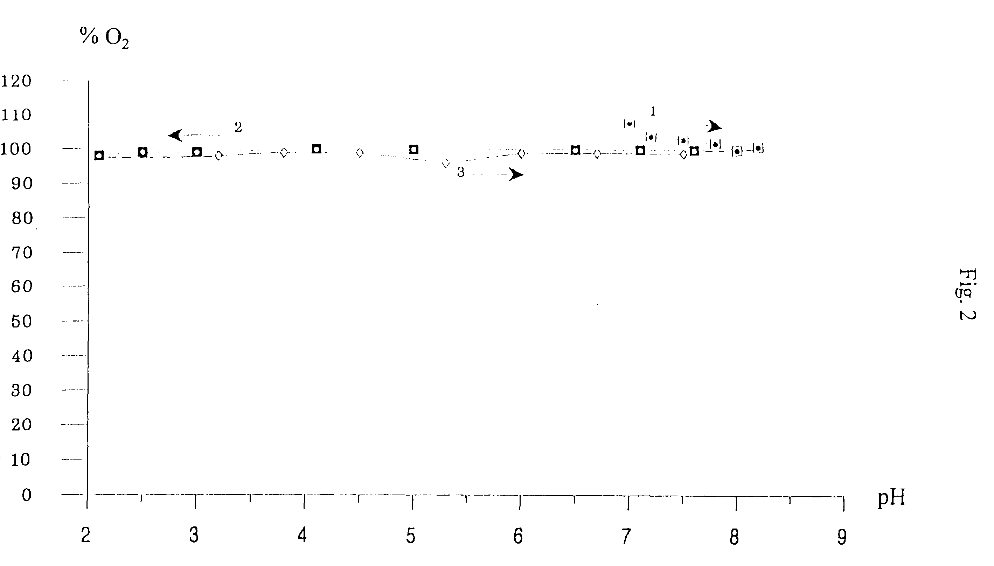

La description ci-après se réfère aux figures annexées qui représentent la libération d'oxygène en fonction du pH pour la SPD (figure 1) et pour un extrait de *Porphyridium cruentum* (figure 2). La *Porphyridium cruentum* est une algue microscopique.

Les courbes présentées sur les figures 1 et 2 ont été tracées en suivant la variation de la teneur en oxygène d'une solution à 0,5 % d'extrait d'algue dans 150 ml de tampon phosphate 0,1 M de pH 7,2 lors de la basification par une solution 5N de soude, puis lors de l'acidification par une solution 5N d'acide chlorhydrique. La teneur en oxygène est mesurée à l'aide d'une électrode à oxygène tandis que le pH est déterminé par une électrode de pH.

On voit nettement sur la figure 1 qu'il y a diminution du dégagement d'oxygène lors de la basification de la solution (courbe 1 sur la figure 1), puis augmentation du dégagement d'oxygène entre pH 7,5 et pH 2 (courbe 2 sur la figure 1) et de nouveau diminution du dégagement d'oxygène lors d'une nouvelle basification (courbe 3 sur la figure 1). Au contraire, sur la figure 2, on observe qu'il n'y a pas de modification du dégagement d'oxygène avec l'extrait de *Porphyridium cruentum ;* les courbes 1, 2, et 3 de la figure 2 sont pratiquement au même niveau.

Ainsi, ces courbes montrent que la SPD utilisée selon l'invention présente la propriété de transporter l'oxygène contrairement à l'extrait de *Porphyridium cruentum.*

Les exemples suivants illustrent l'invention. Dans ces exemples, les proportions indiquées sont des pourcentages en poids.

### Exemple 1 : Crème tonifiante pour le visage

| | |
|---|---|
| SPD | 2 % |
| Stéarate de glycérol | 1,5 % |
| Polysorbate 60 (Tween 60 vendu par la société ICI) | 1 % |
| Huile de vaseline | 24 % |
| Alcool cétylique | 2,5 % |
| Triéthanolamine | qs pH=7 |
| Eau | qsp 100 % |

L'émulsion huile-dans-eau obtenue constitue une crème de couleur beige, apte par une application quotidienne à tonifier la peau.

### Exemple 2 : Gel traitant pour le cuir chevelu

| | |
|---|---|
| SPD | 1 % |
| Minoxidil | 1 % |
| EDTA | 0,07 % |
| Propylène glycol | 5 % |
| Carbomer | 0,5 % |
| Triéthanolamine | qs pH=7 |
| Conservateur | qs |
| Eau | qsp 100 % |

Le gel obtenu peut être appliqué sur le cuir chevelu pour le traitement anti-chute des cheveux.

### Exemple 3 : Crème pour le visage (dispersion vésiculaire)

| *Phase vésiculaire:* | |
|---|---|
| Ether de lauryl polyglycéryl-6-cétéarylglycol (Chimexane NS) | 0,9 % |
| Acylglutamate de sodium (HS21) | 0,1 % |
| Glycérine | 3 % |
| SPD | 0,5 % |
| Eau | 15 % |

| *Phase huileuse :* | |
|---|---|
| Perhydrosqualène | 10 % |

| *Phase aqueuse :* | |
|---|---|
| Acide éthylène tétra-(méthylène)phosphonique (Dequest 2041 vendu par la société Monsanto) (Séquestrant) | 0,05 % |
| Carbomer | 0,4 % |
| Triéthanolamine | qs pH=7 |
| Eau | qsp 100 % |

Cette dispersion vésiculaire est préparée de la façon suivante :
- L'éther de lauryl polyglycéryl-6-cétéarylglycol est ajouté à l'acylglutamate à une température de 100°C ; après un abaissement de la température à 90°C, on ajoute la glycérine et l'eau ;
- on refroidit le mélange à 50°C et on ajoute la SPD, puis on homogénéise 2 fois à l'aide d'un homogénéiseur ;
- le produit obtenu est refroidi rapidement jusqu'à la température ambiante et dilué avec 20 g d'eau. On ajoute alors la phase huileuse puis on homogénéise ; on ajoute ensuite la phase aqueuse préalablement préparée, puis l'ensemble est neutralisé par la triéthanolamine.

On obtient une crème lisse et brillante, utilisable notamment comme crème de soin pour oxygéner et tonifier la peau.

## Revendications

1. Utilisation dans une composition cosmétique comportant un milieu cosmétiquement acceptable, d'au moins un extrait d'au moins une algue macroscopique, de poids moléculaire inférieur à 10000 daltons comme transporteur d'oxygène.

2. Utilisation d'au moins un extrait d'au moins une algue macroscopique, de poids moléculaire inférieur à 10000 daltons comme transporteur d'oxygène pour la fabrication d'une composition dermatologique comportant un milieu dermatologiquement acceptable.

3. Utilisation selon l'une quelconque des revendications précédentes, caractérisée en ce que l'extrait est la SPD ou la Superphyco-D.

4. Utilisation selon l'une quelconque des revendications précédentes, caractérisée en ce que l'extrait est présent en une quantité allant de 0,05 à 30 % en poids par rapport au poids total de la composition.

5. Utilisation selon l'une quelconque des revendications précédentes, caractérisée en ce que la composition contient en outre un actif cosmétique et/ou dermatologique.

6. Procédé cosmétique pour oxygéner la peau et/ou le cuir chevelu, caractérisé en ce que l'on applique sur la peau et/ou le cuir chevelu, une composition cosmétique contenant au moins un extrait d'au moins une algue macroscopique, de poids moléculaire inférieur à 10000 daltons.

7. Procédé cosmétique pour tonifier la peau et/ou le cuir chevelu, caractérisé en ce que l'on applique sur la peau et/ou le cuir chevelu, une composition cosmétique contenant au moins un extrait d'au moins une algue macroscopique, de poids moléculaire inférieur à 10000 daltons.

8. Procédé cosmétique pour empêcher la chute des cheveux, caractérisé en ce que l'on applique sur le cuir chevelu une composition cosmétique contenant au moins un extrait d'au moins une algue macroscopique, de poids moléculaire inférieur à 10000 daltons.

9. Procédé selon l'une quelconque des revendications 6 à 8, caractérisé en ce que l'extrait est la SPD ou la Superphyco-D.

10. Procédé selon l'une quelconque des revendications 6 à 9, caractérisé en ce que l'extrait est présent en une quantité allant de 0,05 à 30 % en poids par rapport au poids total de la composition.
